# EUROPEAN PATENT APPLICATION

(11) **EP 2 241 376 A1**
(43) Date of publication of application: **20.10.2010**
(21) Application number: 09704618.9
(22) Date of filing: 20.01.2009
(51) Int. Cl.: B05B 5/025, B05B 5/08, B03C 3/41, H01T 19/04

(54) **ELECTROSTATIC SPRAY DEVICE**

(30) Priority: 22.01.2008 JP 2008011938
(71) Applicant: Daikin Industries, Ltd., Osaka 530-8323 (JP)
(72) Inventor: INOKUCHI, Yume, Sakai-shi Osaka 591-8511 (JP); OKUMOTO, Mamoru, Sakai-shi Osaka 591-8511 (JP); OBATA, Kouei, Sakai-shi Osaka 591-8511 (JP)
(74) Representative: HOFFMANN EITLE
(86) International application number: PCT/JP2009/000174
(87) International publication number: WO 2009/093437

(57) **Abstract**

An electrostatic sprayer includes: a housing; a container (11) accommodated in the housing and filled with liquid; a nozzle (20) attached to the container (11), with a tip thereof opened to an outside of the container (11) and a rear end thereof opened to an inside of the container (11); a pressurization mechanism for compressing the container (11) to supply the liquid to the tip of the nozzle (20); and a conductive member (12) for applying a predetermined voltage to the liquid in the container (11). The liquid, to which the predetermined voltage is applied by the conductive member (12), is sprayed in an atomized state from the tip of the nozzle (20). The nozzle (20) is formed in a needle shape by a flexible resin member.

## Description

### TECHNICAL FIELD

The present invention relates to an electrostatic sprayer, and more particularly to a structure of a nozzle.

### BACKGROUND ART

Some conventional liquid sprayers spray liquid stored in a container. Among liquid sprayers of this type, an electrostatic sprayer is known in the art that sprays liquid, which has been fed into a nozzle which is the outlet of the container, from the tip of the nozzle by an electric field intensity. Patent Document 1 discloses an electrostatic sprayer of this type. The electrostatic sprayer of Patent Document 1 includes a housing including a pair of cases that are hinged together at one end. Each case includes a pad formed by an elastically-deformable material. With this electrostatic sprayer, when the housing is closed, the pads contract while sandwiching therebetween a small bag which is a container for storing a material. The small bag is compressed by the pads, and the liquid in the small bag is supplied into a nozzle. An electric field is formed at the tip of the nozzle in this state to thereby spray the liquid in the small bag from the tip of the nozzle.

### CITATION LIST

### PATENT DOCUMENT

PATENT DOCUMENT 1: Japanese Published Patent Application No. H05-138081 SUMMARY OF THE INVENTION

### TECHNICAL PROBLEM

However, if the nozzle is formed in a thin needle shape in the conventional electrostatic sprayer, the user, or the like, may be injured if the user, or the like, inadvertently touches the tip of the nozzle, etc.

The present invention has been made in view of the above, and has an object to form a flexible nozzle.

### SOLUTION TO THE PROBLEM

A first aspect is directed to an electrostatic sprayer including: a housing (41); a container (11) accommodated in the housing (41) and filled with liquid; a nozzle (20) attached to the container (11), with a tip thereof opened to an outside of the container (11) and a rear end thereof opened to an inside of the container (11); a pressurization device (50) for compressing the container (11) to supply the liquid to the tip of the nozzle (20); and a voltage application device (12) for applying a predetermined voltage to the liquid in the container (11), wherein the liquid, to which the predetermined voltage is applied by the voltage application device (12), is sprayed in an atomized state from the tip of the nozzle (20), and the nozzle (20) is formed in a needle shape by a flexible resin member.

According to the first aspect, even if the user, or the like, touches the nozzle (20), the nozzle (20), which is formed in a needle shape by a flexible resin member, bends. On the other hand, the voltage application device (12) electrically charges the liquid in the container (11). When the container (11) is compressed in such a state, the electrically-charged liquid flows into the nozzle (20), and blows out as minute particles of liquid from the tip of the nozzle (20).

A second aspect is according to the first aspect, wherein the nozzle (20) includes a free end (21a) formed to a predetermined length from a base of the nozzle (20) to the tip thereof.

According to the second aspect, even if the user, or the like, touches the nozzle (20), the nozzle (20), which includes the free end (21a) formed to a predetermined length, deforms. On the other hand, the voltage application device (12) electrically charges the liquid in the container (11). When the container (11) is compressed in such a state, the electrically-charged liquid flows into the nozzle (20), and blows out as minute particles of liquid from the tip of the nozzle (20).

A third aspect is according to the first or second aspect, wherein the free end (21a) of the nozzle (20) is formed to a length of 6 mm or more.

According to the third aspect, even if the user touches the nozzle (20), the nozzle (24), which includes the free end (21a) formed to be 6 mm or more, deforms. On the other hand, the voltage application device (12) electrically charges the liquid in the container (11). When the container (11) is compressed in such a state, the electrically-charged liquid flows into the nozzle (20), and blows out as minute particles of liquid from the tip of the nozzle (20).

A fourth aspect is according to one of the first to third aspects, wherein the nozzle (20) is formed with an outer diameter size of 0.5 mm or less and an inner diameter size of 0.2 mm or less.

According to the fourth aspect, even if the user, or the like, touches the nozzle (20), the nozzle (20), which is formed with an outer diameter size of 0.5 mm or less and an inner diameter size of 0.2 mm or less, bends. On the other hand, the voltage application device (12) electrically charges the liquid in the container (11). When the container (11) is compressed in such a state, the electrically-charged liquid flows into the nozzle (20), and blows out as minute particles of liquid from the tip of the nozzle (20).

A fifth aspect is according to one of the first to fourth aspects, the nozzle (20) is formed by a polyetherimide resin.

According to the fifth aspect, even if the user, or the like, touches the nozzle (20), the nozzle (20), which is formed by a polyetherimide resin, bends. On the other hand, the voltage application device (12) electrically charges the liquid in the container (11). When the container (11) is compressed in such a state, the electrically-charged liquid flows into the nozzle (20), and blows out as minute particles of liquid from the tip of the nozzle (20).

### ADVANTAGES OF THE INVENTION

According to the aspect above, since the nozzle (20) is formed in a needle shape by a flexible resin member, the nozzle (20) can bend easily. Therefore, even if the user, or the like, touches the nozzle (20) inadvertently, the nozzle (20) deforms, and it is therefore possible to reliably prevent the user, or the like, from being injured.

According to the second aspect, since the free end (21a) is formed to a predetermined length from the base of the nozzle (20) to the tip thereof, the free end (21a) of the nozzle (20) can deform easily. Therefore, even if the user, or the like, touches the nozzle (20) inadvertently, the free end (21a) of the nozzle (20) deform, and it is therefore possible to reliably prevent the user, or the like, from being injured.

According to the third aspect, since the free end (21a) of the nozzle (20) is formed to a length of 6 mm or more, the free end (21a) of the nozzle (20) can defonn easily. Therefore, even if the user, or the like, touches the nozzle (20) inadvertently, the free end (21a) of the nozzle (20) deforms, and it is therefore possible to reliably prevent the user, or the like, from being injured.

According to the fourth aspect, since the nozzle (20) is formed with an outer diameter size of 0.5 mm or less and an inner diameter size of 0.2 mm or less, the nozzle (20) can bend easily. Therefore, even if the user, or the like, touches the nozzle (20) inadvertently, the nozzle (20) deforms, and it is therefore possible to reliably prevent the user, or the like, from being injured.

According to the fifth aspect, since the nozzle (20) is formed by a polyetherimide resin, the nozzle (20) can be formed to be flexible. Therefore, even if the user, or the like, touches the nozzle (20) inadvertently, the nozzle (20) deforms, and it is therefore possible to reliably prevent the user, or the like, from being injured.

### BRIEF DESCRIPTION OF THE DRAWINGS

[FIG. 1] FIG. 1 is a perspective view showing an electrostatic sprayer as viewed from a first cover side of the present embodiment.
[FIG. 2] FIG. 2 is a perspective view showing the electrostatic sprayer as viewed from a second cover side of the present embodiment.
[FIG. 3] FIG. 3 is a front view showing the electrostatic sprayer of the present embodiment.
[FIG. 4] FIG. 4 is a front view showing the electrostatic sprayer of the present embodiment in a power-off (non-use) state.
[FIG. 5] FIG. 5 is a schematic vertical cross-sectional view showing the electrostatic sprayer of the present embodiment.
[FIG. 6] FIG. 6 is a schematic vertical cross-sectional view showing the electrostatic sprayer of the present embodiment.
[FIG. 7] FIG. 7 is a schematic view showing a configuration of a spray cartridge of the present embodiment.
[FIG. 8] FIG. 8 is a schematic view showing a configuration of a spray cartridge of the present embodiment.

### DESCRIPTION OF REFERENCE CHARACTERS

- 11: Container
- 12: Conductive member
- 20: Nozzle
- 21a: (Nozzle) tip portion
- 41: Housing
- 50: Pressurization mechanism

### DESCRIPTION OF EMBODIMENTS

An embodiment of the present invention will now be described with reference to the drawings.

The present embodiment is an application of the present invention to an electrostatic sprayer.

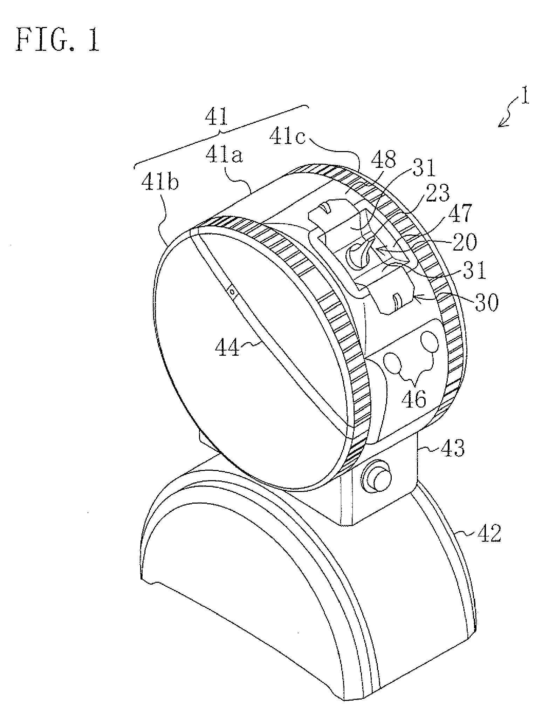

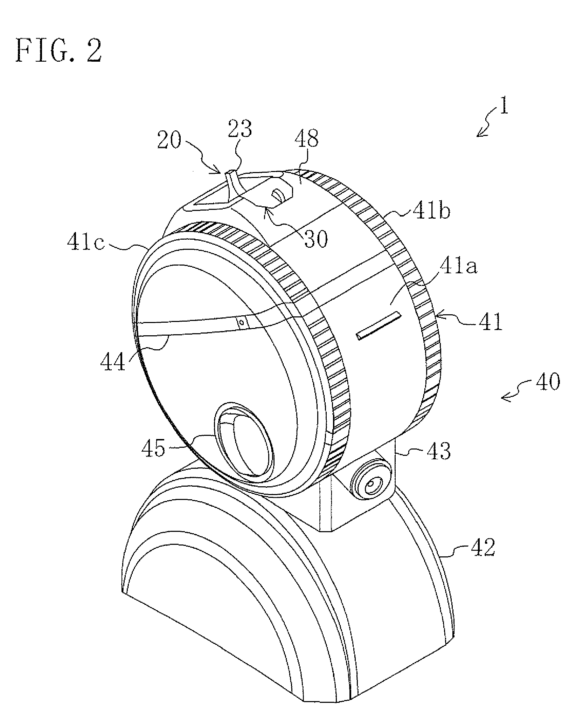

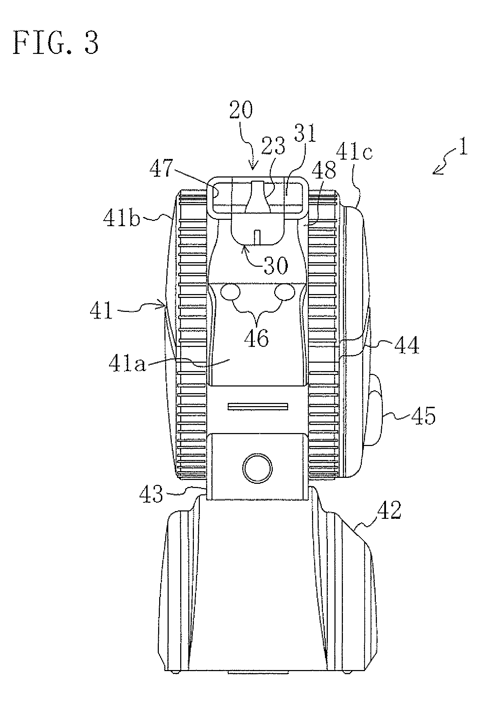

As shown in FIGS. 1-6, an electrostatic sprayer (1) of the present embodiment is used while placed on a table, or the like. The electrostatic sprayer (1) includes a housing (41) and a spray cartridge (10). In the electrostatic sprayer (1), the spray cartridge (10) can be attached to/detached from the housing (41).

The spray cartridge (10) includes a container (11) formed as a flat bag-like container, a conductive member (12) and a nozzle (20) inserted into the container (11), and a nozzle cap (23) for preventing the liquid in the nozzle (20) from drying. Note that the details of the spray cartridge (10) will be described later.

The housing (41) includes a housing main body (41a) and a pair of cover members (41b, 41c) covering the opposite ends of the housing main body (41a), and a base cover (42) is attached to the housing main body (41a) covering the side surface thereof. A pressurization mechanism (50), which is the pressurization device, and a power supply section

(not shown) are accommodated in the housing (41).

The housing main body (41a) is formed in a cylindrical shape, and a supporting portion (43) supporting the housing main body (41a) is provided on a lower portion thereof, with a shroud (48) protecting the nozzle (20) formed on an upper portion thereof. The shroud (48) bulges outwardly from the housing main body (41a), with a depressed surrounding portion formed generally in a ventral portion thereof. The shroud (48) includes a hole portion for the attachment of the spray cartridge (10). LEDs (46) (two in FIG. 1) are attached below the hole portion in the upper portion of the housing main body (41a). The LEDs (46) illuminate the liquid sprayed from the tip of the nozzle (20) so that the user of the electrostatic sprayer (1) is allowed to check how it is being sprayed. Although not shown in the figures, one end of a constant-load spring (51) to be described later is attached to the inside of the housing main body (41a).

The cover members (41b, 41c) include a first cover (41b) and a second cover (41c), together forming a pair. First, the first cover (41b) is formed in a circular shape of generally the same outer shape as the first end surface of the housing main body (41a), and is attached so as to cover the first end surface of the housing main body (41a). The first cover (41b) is attached so that the user can turn the first cover (41b) in the circumferential direction of the housing main body (41a). A strip-shaped counter electrode (44) for forming an electric field with electrically-charged liquid is provided on the front surface of the first cover (41b), and a cylindrical roll-up portion whose diameter is smaller than that of the first cover (41b) is formed on the back surface of the first cover (41b) though not shown in the figures. The roll-up portion includes a spiral-shaped notch cut along the circumferential direction of the roll-up portion, thereby restricting the movement of a pressurization stage (52) to be described later. Next, the second cover (41c) is formed in a circular shape of generally the same outer shape as the second end surface of the housing main body (41a), and is attached so as to cover the second end surface of the housing main body (41a). On the front surface of the second cover (41c), the strip-shaped counter electrode (44) for forming an electric field with electrically-charged liquid is provided, and a volume knob (45) that operates in connection with the output adjusting volume of the power supply section to be described later is provided.

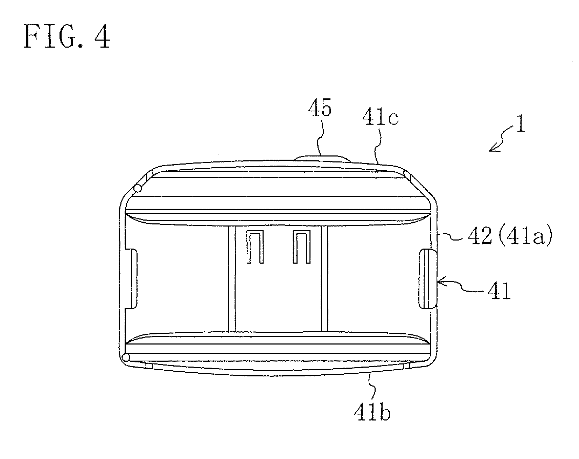

The base cover (42) protects the nozzle (20) in a power-off state (non-use state) of the electrostatic sprayer (1), whereas it is used as a base for supporting the housing (41) while in use. The base cover (42) is formed in a bowl shape conforming to the cylindrical side surface of the housing main body (41a). The base cover (42) is attached to the housing (41) covering the hole portion on the upper side surface of the housing main body (41a) as shown in FIG. 4 in a power-off state (non-use state), whereas it is detached from the housing (41) and attached to the supporting portion (43) on the lower side of the housing main body (41a) while in use. Note that the electrostatic sprayer (1) can be adjusted between two different heights, one where the base cover (42) is attached to the lower portion of the supporting portion (43) and the housing (41) is supported by the base cover (42) from below, and another where the housing (41) is supported only by the supporting portion (43) from below.

The pressurization mechanism (50) is for compressing the container (11) of the spray cartridge (10) to carry the liquid in the container (11) to the tip of the nozzle (20). The pressurization mechanism (50) includes the constant-load spring (51), the pressurization stage (52), and a partition plate (53). Specifically, inside the housing (41), the constant-load spring (51) of the pressurization mechanism (50) moves the pressurization stage (52) toward the second cover (41c) so as to compress the container (11) by sandwiching it between the pressurization stage (52) and the partition plate (53).

The pressurization stage (52) is formed in a cylindrical shape with a bottom, and includes, on the opposite edge portions, spring holding portions (52a) to which the constant-load springs (51) are attached. Although not shown in the figures, the inner surface of the pressurization stage (52) includes a protruding portion that is protruding toward the center of the pressurization stage (52). When the first cover (41b) is turned in the circumferential direction of the housing main body (41a) with the protruding portion in contact with the notch formed in the roll-up portion of the first cover (41b), the protruding portion is guided toward the first cover (41b) along the spiral notch. This in turn moves the pressurization stage (52) toward the first cover (41b), thereby holding the pressurization stage (52) by the first cover (41b). In this state, the container (11) of the spray cartridge (10) is spaced apart from the pressurization stage (52). Then, when the first cover (41b) is turned in the reverse direction to thereby guide the protruding portion toward the second cover (41 c) along the spiral notch, the protruding portion comes off the roll-up portion. At this point, the pressurization stage (52) can be moved with respect to the first cover (41b). That is, the configuration is such that the pressurization stage (52) can be held or released by turning the first cover (41b).

The constant-load spring (51) is a spring obtained by winding in a spiral pattern a strip-shaped metal plate formed with a constant curvature. The constant-load spring (51) is **characteristic in that** once the stroke exceeds a predetermined value, the restoring force stays constant even if the stroke further increases. The main body of the constant-load spring (51) is attached to the spring holding portion (52a) of the pressurization stage (52), and one end of the metal plate wound in a spiral pattern is attached to the inside of the housing main body (41a).

The partition plate (53) is formed by a plate member having a flat plate shape, and is placed in the housing (41) so as to oppose the pressurization stage (52) with the container (11) of the spray cartridge (10) being interposed therebetween. The partition plate (53) is attached to the housing main body (41a).

Although not shown in the figures, the power supply section is provided closer to the second cover (41c) with respect to the partition plate. The power supply section transduces the output voltage from the battery accommodated in the housing (41) to a high voltage of 6 kV, for example. The transduced high voltage is applied to the liquid in the container (11) of the spray cartridge (10) via the conductive member (12). That is, the conductive member (12) formes the voltage application device. The power supply section is provided with an output adjusting volume for adjusting the output voltage, and the output adjusting volume is configured so that it is turned in connection with the volume knob (45) attached to the second cover (41c). That is, it is configured so that the output voltage from the power supply section can be adjusted as necessary by turning the volume knob (45). Note that the power supply section may be configured so that the voltage is transduced to a value that is 0 kV or more and 12 kV or less.

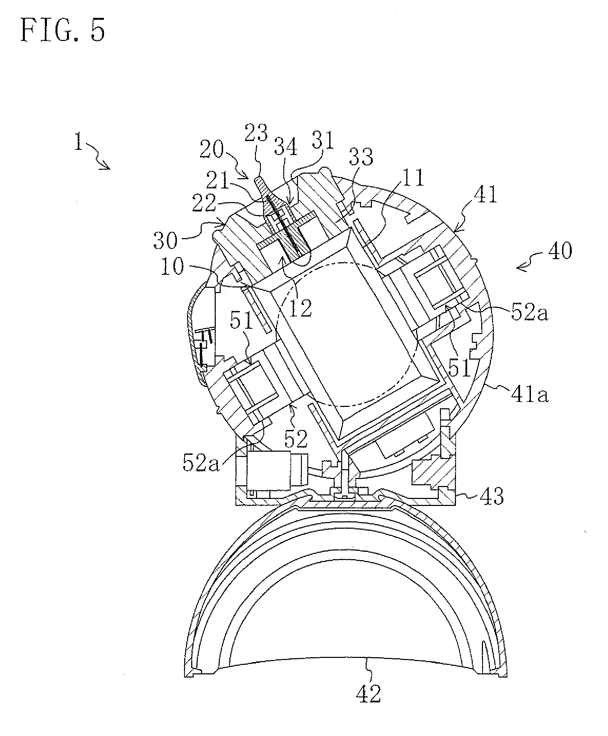

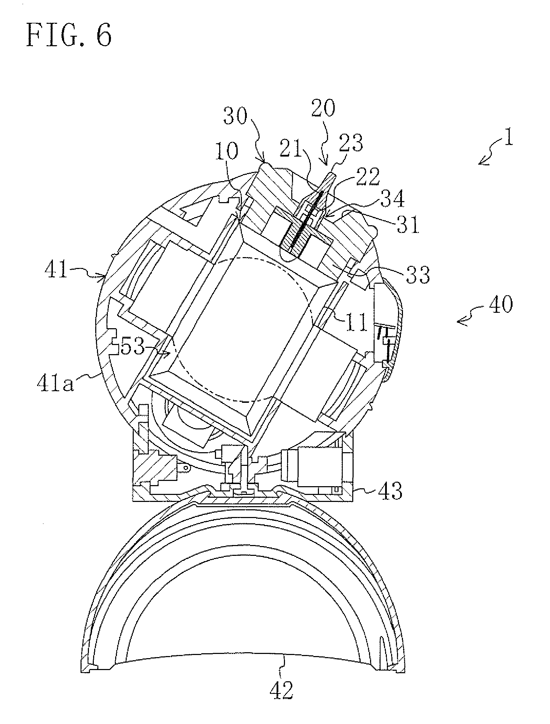

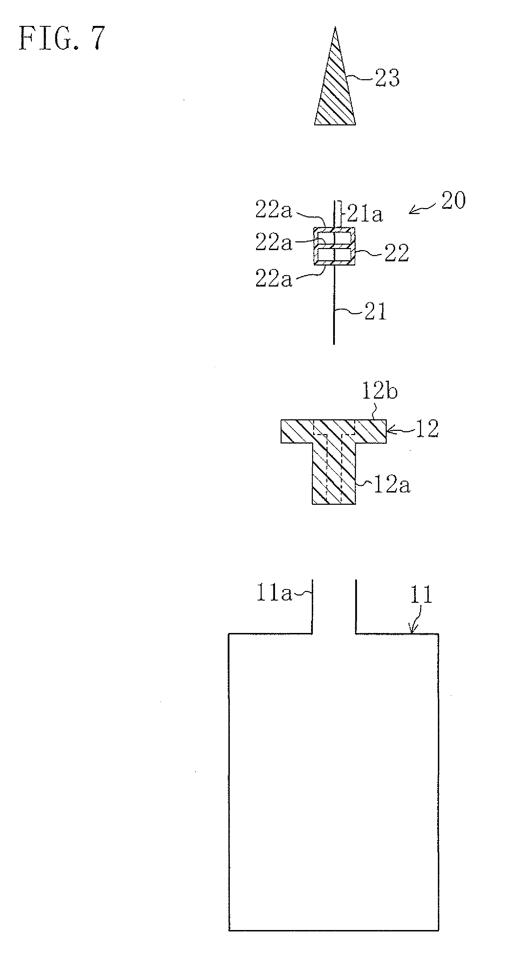

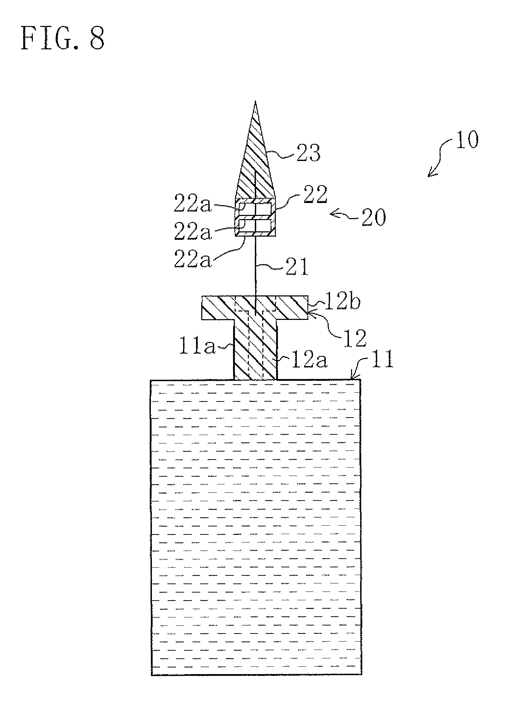

As shown in FIGS. 7 and 8, the spray cartridge (10) includes the container (11) formed as a flat bag-like container, the conductive member (12) and the nozzle (20) inserted into the container (11), and the nozzle cap (23) for preventing the liquid in the nozzle (20) from drying. As shown in FIGS. 5 and 6, the spray cartridge (10) is accommodated in the housing (41), with a nozzle base (30) for protecting the nozzle (20) attached thereto. It is adjusted so that the tip of the nozzle (20) points upward when the spray cartridge (10) is accommodated in the housing (41). The spray cartridge (10) is configured so that the liquid in the container (11) is supplied into the nozzle (20) as the container (11) is compressed. Note that the spray cartridge (10) is replaced when there is little liquid left in the container (11).

The container (11) is formed by putting together two rectangular sheets formed by a liquid-impermeable, relatively flexible material. The two rectangular sheets are bonded together along the four sides thereof, thus forming a hollow container. In the central portion on the upper surface of the container (11), a container tip portion (11a) for connecting the inside and the outside of the container (11) with each other is formed protruding toward the outside of the container (11). The container tip portion (11a) is formed in a hollow cylindrical shape, into which the conductive member (12) to be described later and the nozzle (20) are inserted. The inside of the container (11) is filled with a liquid containing a moisturizing ingredient and an antioxidant ingredient. The concentration of the liquid is adjusted so that the electrical resistivity thereof is within a range of 10×10⁴ Ωcm or more and 1.0×10⁷ Ωcm or less. The container (11) is held in the housing (41) in a position inclined along with the nozzle (20).

The conductive member (12) is for giving an electrical charge to the liquid charged in the container (11). The conductive member (12) is formed by a conductive resin, and is in contact with the liquid in the container (11) while being connected to the power supply section. Specifically, the conductive member (12) includes an insertion portion (12a) inserted into the container tip portion (11a), and a flange (12b) formed at one end of the insertion portion (12a).

The insertion portion (12a) is formed in a cylindrical shape, with the nozzle (20) running through generally the central portion thereof. The insertion portion (12a) is inserted into the container tip portion (11a), with the lower end thereof in contact with the liquid in the container (11).

The flange (12b) is formed in a large-diameter circular shape, and is attached to the upper end of the insertion portion (12a). The flange (12b) includes a depressed portion, with which the lower end of a nozzle holding portion (22) to be described later engages, formed generally in the central portion of the upper end surface of the flange (12b), with the nozzle (20) running through generally the central portion of the flange (12b). That is, the nozzle (20) is running through the conductive member (12) from the lower end of the insertion portion (12a) to the upper end of the flange (12b). While the insertion portion (12a) of the conductive member (12) inserted in the container tip portion (11a), the lower end surface of the flange (12b) shuts the opening at the tip of the container tip portion (11a) so that the liquid is sealed in the container (11).

The nozzle base (30) forms a portion of the shroud (48). The nozzle base (30) is formed in an oblong, generally rectangular parallelepiped shape, with a though hole (34) in the central portion thereof, from which the tip of the nozzle (20) protrudes. At opposite ends of the nozzle base (30) in the longitudinal direction thereof, wall portions (31) are formed each extending upward, and leg portions (33) are formed extending downward. When the nozzle base (30) is attached to the spray cartridge (10), the nozzle (20) is fixed while protruding through the though hole (34). Here, the wall portion (31) is formed so as to protrude to a height greater than or equal to the height of the tip of the nozzle (20). When the nozzle base (30), attached to the spray cartridge (10), is attached the housing (41), the wall portions (31) form the opposite ends of the shroud (48) in the longitudinal direction thereof. That is, the wall portion (31) of the nozzle base (30) and an inner surface (47) of the shroud (48) form a continuous wall surface, surrounding the protruding nozzle (20). This continuous wall surface is formed spaced apart from the tip of the nozzle (20) by a distance (1 cm in the present embodiment) such that the electric field formed around the nozzle (20) is not interfered. Note that the height of the inner surface (47) of the shroud (48) is generally equal to the height of the wall portion (31) of the nozzle base (30).

The nozzle cap (23) is for hermetically closing the tip portion of the nozzle (20) to thereby prevent the liquid left inside the nozzle (20) from drying in a power-off (non-use) state of the electrostatic sprayer (1). The nozzle cap (23) includes a large-diameter portion covering the nozzle holding portion (22), and a small-diameter portion covering the tip portion of a nozzle main body (21). Note that the nozzle holding portion (22) and the nozzle main body (21) will be described later.

The large-diameter portion is formed in a cylindrical shape that is slightly larger than the nozzle holding portion (22), covering the nozzle holding portion (22). The small-diameter portion is formed in a cylindrical shape that is smaller than the large-diameter portion and slightly larger than the tip portion of the nozzle main body (21), covering the tip portion of the nozzle main body (21). The inner surface of the small-diameter portion is formed in a tapered surface that narrows from the base end toward the distal end. That is, when the nozzle (20) is inserted into the nozzle cap (23), the tip of the nozzle main body (21) is guided from the base end to the tip along the tapered surface of the small-diameter portion of the nozzle cap (23). It is fixed as the nozzle holding portion (22) abuts the upper end surface of the large-diameter portion of the nozzle cap (23).

Next, a configuration of the nozzle (20) will be described with reference to FIGS. 7 and 8.

The nozzle (20) is for discharging to the outside the liquid charged in the container (11). The nozzle (20) includes the nozzle main body (21), which is a thin tube, and the nozzle holding portion (22) formed integral with the nozzle main body (21).

The nozzle main body (21) is formed in a tube shape having a uniform inner diameter from the rear end to the tip thereof, with the rear end opened inside the container (11) and the tip opened outside the housing (41). The nozzle main body (21) is formed with an outer diameter of 0.35 mm and an inner diameter of 0.1 mm. The nozzle main body (21) is formed in a needle shape by polyetherimide (PEI) which is a flexible resin material, for example, and is inserted into the conductive member (12). The nozzle main body (21) includes a tip portion (21a) which is a free end of 6-7 mm extending from the upper end surface of the nozzle holding portion (22) to the tip of the nozzle main body (21). Note that the nozzle main body (21) may be provided with a resistor serving as a passage resistance for the liquid moving toward the tip in order to adjust the amount of liquid per unit time passed from the container (11).

The nozzle holding portion (22) is for holding the nozzle main body (21) formed by a flexible resin material while fixing it to the container (11). The nozzle holding portion (22) is formed by a generally cylindrical resin member having a larger diameter than the nozzle main body (21), and includes leg portions (22a) formed in an upper end portion, a lower end portion and a central portion, spaced apart from one another, to be in contact with the circumferential surface of the nozzle main body (21), thus supporting the nozzle main body (21) at three points. Note that the leg portions (22a) are formed with a size of 1.5 mm in the width direction thereof. The nozzle holding portion (22) is formed by insert molding with the nozzle main body (21) running through the center of the nozzle holding portion (22). The lower end of the nozzle holding portion (22) is engaged with the depressed portion formed on the upper end surface of the flange (12b) of the conductive member (12). That is, the nozzle holding portion (22) forms a base portion of the nozzle (20).

### -Operation-

Next, an operation of the electrostatic sprayer (1) of the present embodiment will be described. The electrostatic sprayer (1) performs so-called cone-jet mode EHD spraying.

The electrostatic sprayer (1) is brought to an operable state when the user inserts the spray cartridge (10) into the housing (41). At this point, there is a load generated from the constant-load spring (51) acting upon the pressurization stage (52).

First, the user removes the nozzle cap (23) from the nozzle (20). Then, when the user manually turns the first cover (41b) in the circumferential direction of the housing main body (41a), the restriction of the first cover (41b) on the pressurization stage (52) is removed. When the restriction is removed, the spring force of the constant-load spring (51) is applied to the pressurization stage (52), and the pressurization stage (52) moves toward the partition plate (53). The container (11) filled with the liquid is compressed by the pressurization stage (52), which has moved, and the partition plate (53). The liquid in the compressed container (11) flows into the inside of the nozzle main body (21). Then, the liquid, which has flown in to the inside of the nozzle main body (21), moves to the tip of the nozzle main body (21). On the other hand, since an electrical charge is applied to the liquid in the container (11) from the power supply section via the conductive member (12), the electrically-charged liquid is therefore polarized, and the + (positively)-charged liquid is drawn together to the vicinity of the gas-liquid interface at the tip of the nozzle main body (21). Then, at the tip of the nozzle main body (21), the gas-liquid interface is drawn into a conical shape by the potential difference with respect to the counter electrode (44), and a portion of the aqueous solution is ripped off of the apex of the conically-shaped gas-liquid interface to form a droplet. Note that with the level of the application voltage and the electrical resistivity of the liquid of the present embodiment, the size of the droplet flying off the tip of the nozzle main body (21) is generally in a range of 50 µm to 200 µm. The liquid flying off the nozzle main body (21) reaches a distance of about 40-50 cm away from the tip of the nozzle main body (21). If the user places the electrostatic sprayer (1) about 50 cm in front of the user, with the tip of the nozzle main body (21) pointing toward the face of the user, the flying droplets attach to the face of the user. Then, when the user manually turns the first cover (41b) in the reverse direction of the housing main body (41a), the pressurization stage (52) is restricted by the first cover (41b), and the voltage from the power supply section is stopped, thus stopping the spray. Finally, the user puts the nozzle cap (23) on the nozzle (20).

### -Advantages of Embodiment-

According to the embodiment above, the nozzle main body (21) is formed by a polyetherimide resin with an outer diameter size of 4.35 mm and an inner diameter size of 0.1 mm. Therefore, the nozzle main body (21) is formed so that it can bend easily and is flexible. Since the free end (21a) of the nozzle main body (21) is 6-7 mm, the free end (21a) of the nozzle main body (21) can deform easily. Therefore, even if the user, or the like, touches the nozzle (20) inadvertently, the free end (21a) of the nozzle main body (21) deforms, and it is therefore possible to reliably prevent the user, or the like, from being injured.

### <Alternative Embodiment>

The present invention may have the following configuration for the embodiment above.

The nozzle main body (21) of the embodiment above may be formed with an outer diameter of 0.50 mm and an inner diameter of 0.2 mm. In the embodiment above, the liquid to be sprayed may be an aqueous solution containing hyaluronic acid, or an aqueous solution of theanine. Alternatively, it may be an aqueous solution of an antioxidant such as catechin or proanthocyanidin. It may be a liquid containing a material having a function of suppressing the propagation of microorganisms or a function of annihilating microorganisms, or a liquid containing a material that deodorizes odor molecules in the air through a chemical change based on neutralization, or the like. It may be a liquid containing any of various perfumes, noxious insect repellant, or the like.

Note that the embodiment described above is essentially a preferred embodiment, and is not intended to limit the scope of the present invention, the applications thereof, or the uses thereof.

### INDUSTRIAL APPLICABILITY

As describe above, the present invention is useful for an electrostatic sprayer having a nozzle.

## Claims

1. An electrostatic sprayer comprising:
a housing (41);
a container (11) accommodated in the housing (41) and filled with liquid;
a nozzle (20) attached to the container (11), with a tip thereof opened to an outside of the container (11) and a rear end thereof opened to an inside of the container (11);
a pressurization device (50) for compressing the container (11) to supply the liquid to the tip of the nozzle (20); and
a voltage application device (12) for applying a predetermined voltage to the liquid in the container (11), wherein
the liquid, to which the predetermined voltage is applied by the voltage application device (12), is sprayed in an atomized state from the tip of the nozzle (20), and
the nozzle (20) is formed in a needle shape by a flexible resin member.

2. The electrostatic sprayer of claim 1, wherein
the nozzle (20) includes a free end (21a) formed to a predetermined length from a base of the nozzle (20) to the tip thereof.

3. The electrostatic sprayer of claim 1 or 2, wherein
the free end (21a) of the nozzle (20) is formed to a length of 6 mm or more.

4. The electrostatic sprayer of any of claims 1-3, wherein
the nozzle (20) is formed with an outer diameter size of 0.5 mm or less and an inner diameter size of 0.2 mm or less.

5. The electrostatic sprayer of any of claims 1-4, wherein
the nozzle (20) is formed by a polyetherimide resin.
